(19) Europäisches Patentamt
European Patent Office
Office européen des brevets

(11) Veröffentlichungsnummer: **0 212 605**
**A2**

# (12) EUROPÄISCHE PATENTANMELDUNG

(21) Anmeldenummer: **86111382.7**

(22) Anmeldetag: **18.08.86**

(51) Int. Cl.⁴: **C 07 D 249/08**, C 07 D 233/60, C 07 D 409/06, C 07 D 409/08, A 61 K 31/41, A 61 K 31/415, A 01 N 43/50, A 01 N 43/653

(30) Priorität: **29.08.85 DE 3530799**

(43) Veröffentlichungstag der Anmeldung: **04.03.87 Patentblatt 87/10**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Schaper, Wolfgang, Dr., Rauenthaler Weg 18, D-6000 Frankfurt am Main 71 (DE)**
Erfinder: **Raether, Wolfgang, Dr., Falkensteinstrasse 6, D-6072 Dreieich (DE)**
Erfinder: **Dittmar, Walter, Dr., Uhlandstrasse 10, D-6238 Hofheim am Taunus (DE)**
Erfinder: **Sachse, Burkhard, Dr., An der Ziegelei 30, D-6233 Kelkheim (Taunus) (DE)**
Erfinder: **Hartz, Peter, Dr., An der Zeigelei 28, D-6233 Kelkheim (Taunus) (DE)**

## (54) Azolyl-cyclopropyl-ethanol-Derivate, Verfahren zu ihrer Herstellung und ihre Verwendung.

(57) Azolderivate der Formel I mit A gleich CH oder N und $R^1/R^2$ substituiertes oder unsubstituiertes Phenyl, Biphenyl, Phenoxyphenyl, Benzylphenyl, Benzyloxyphenyl, Naphthyl, Thienyl, Furyl oder Pyridyl sowie deren Salze und Ester, in allen stereoisomeren Formen haben hervorragende antimykotische und jungizide Wirksamkeit.

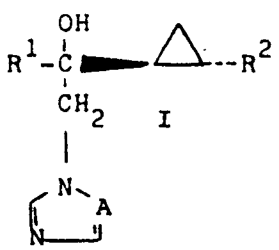

I

Sie werden erhalten durch Umsetzung einer Verbindung II

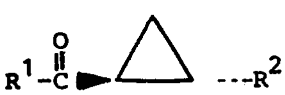

mit einem Schwefelylid der Formel III $(CH_3)_2\underset{(O)_z}{\overset{}{S}}CH_2$ zur Verbindung IV 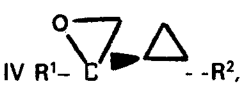 welche mit der entsprechenden Verbindung V 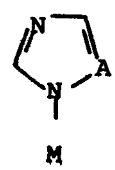 zu I umgesetzt wird.

Man kann auch von Verbindung VI $R^1-\underset{O}{\underset{\|}{C}}-CH=CH-R^2$ ausgehen, diese mit 2 Äquivalenten IIIa $(CH_3)_2\underset{O}{\underset{\|}{S}}CH_2$ umsetzen, wobei Verbindung IV entsteht; oder Verbindung VI wird zunächst mit einem Äquivalent IIIa zu II umgesetzt, und II dann ohne Aufarbeitung mit dem Schwefelylid IIIb $(CH_3)_2SCH_2$ zu IV, diese mit V zu I umgesetzt.

EP 0 212 605 A2

ACTORUM AG

# Azolyl-cyclopropyl-ethanol-Derivate, Verfahren zu ihrer Herstellung und ihre Verwendung

Die Erfindung betrifft Azolyl-cyclopropyl-ethanol-Derivate, Verfahren zu ihrer Herstellung, sowie solche Azole enthaltende pharmazeutische Zusammensetzungen und ihre Verwendung als Pharmazeutika, insbesondere als Antimykotika und Pflanzenschutzmittel, z.B. als Fungizide und Wachstumsregulatoren.

In der US-Patentschrift 4 432 989 werden 1-Aryl-1-cyclopropyl-2-azolyl-ethanole und 1-Aryl-1-alkylcyclopropyl-2-azolyl-ethanole als Fungizide im Pflanzenschutz beschrieben. Jedoch sind darin weder antimykotische Wirkungen beschrieben, noch die erfindungsgemäßen Verbindungen.

Es wurde nun gefunden, daß die erfindungsgemäßen Verbindungen, die sich von den oben erwähnten Verbindungen wesentlich durch die Art der Substitution am Cyclopropanring unterscheiden, sehr gute antimikrobielle, insbesondere antimykotische Eigenschaften aufweisen. Sie sind deshalb zur Bekämpfung von Pilzen bei Mensch und Tier geeignet. Durch fungizide und wachstumsregulierende Eigenschaften sind sie auch als Pflanzenschutzmittel geeignet.

Die Erfindung betrifft daher Azolderivate der Formel (I)

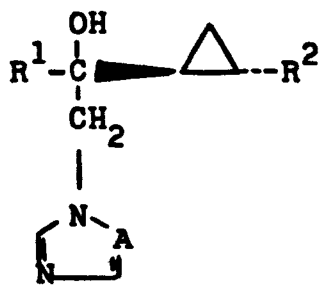

(I)

worin

A CH oder N bedeutet und

$R^1$ und $R^2$ gleich oder verschieden sind und jeweils Phenyl, Biphenyl, Phenoxyphenyl, Benzylphenyl, Benzyloxyphenyl, Naphthyl, Thienyl, Furyl oder Pyridyl bedeuten, wobei die Ringe unsubstituiert oder durch einen, zwei oder drei Substituenten, die gleich oder verschieden sind und jeweils Fluor, Chlor, Brom, Jod, $(C_1-C_8)$-Alkyl, unverzweigt oder verzweigt, unsubstituiert oder durch 1-9 Fluor- oder Chloratome substituiert, $(C_3-C_6)$-Cycloalkyl, $(C_3-C_6)$-Cycloalkyl-$(C_1-C_4)$-alkyl, $(C_1-C_8)$-Alkoxy, $(C_1-C_8)$-Alkylthio oder Nitro bedeuten, substituiert sind,

sowie deren pharmazeutisch akzeptablen Salze und Ester und ihre Stereoisomeren.

Bevorzugt sind Verbindungen der Formel I, worin $R^1$ und $R^2$ jeweils gleich oder verschieden sind und unsubstituiertes Biphenyl, Phenoxyphenyl, Benzylphenyl, Benzyloxyphenyl, Naphthyl, Thienyl, Furyl oder Pyridyl oder eine unsubstituierte oder mit einem oder zwei Substituenten versehene Phenylgruppe bedeuten und diese Substituenten jeweils Fluor, Chlor, Brom, $(C_1-C_4)$-Alkyl, Trifluormethyl, $(C_1-C_4)$-Alkoxy, $(C_1-C_4)$-Alkylthio oder Nitro bedeuten.

Besonders bevorzugt sind Verbindungen der Formel I, worin $R^1$ und $R^2$ die Phenylgruppe bedeutet, welche unsubstituiert oder durch einen oder zwei Substituenten substituiert ist, die Fluor, Chlor, Brom oder die Trifluormethylgruppe bedeuten.

Insbesondere sind Verbindungen I bevorzugt, worin die Substituenten von $R^1$ und $R^2$ jeweils Fluor oder Chlor bedeuten.

Die als Substituenten oder in Zusammenhang mit anderen Substituenten auftretenden $(C_1-C_8)$- bzw. $(C_1-C_4)$-Alkylgruppen

können unverzweigt oder verzweigt sein und beispielsweise die Methyl-, Ethyl-, Propyl-, 1-Methyl-ethyl-, Butyl-, 2-Methylpropyl-, 1,1-Dimethyl-ethyl-, Pentyl-, Hexyl-, Heptyl- oder Octylgruppe bedeuten; die durch Fluor oder Chlor substituierten Alkylgruppen können beispielsweise die Trifluormethyl-, Trichlormethyl, 1,1,2,2-Tetrafluorethyl oder die Nonafluorbutylgruppe bedeuten; die ($C_3$-$C_6$)-Cycloalkylgruppen können die Cyclopropyl-, Cyclobutyl, Cyclopentyl- oder Cyclohexylgruppe bedeuten; die ($C_3$-$C_6$)-Cycloalkyl($C_1$-$C_4$)-alkylgruppen können beispielsweise die Cyclopropylmethyl-, Cyclopentylmethyl-, Cyclohexylmethyl- oder Cyclohexylethylgruppe bedeuten.

Die vorliegende Erfindung betrifft die Verbindungen (I) in Form der freien Base oder eines Säureadditionssalzes oder eines physiologisch hydrolysierbaren und akzeptablen Derivates.

Beispiele pharmazeutisch akzeptabler salzbildender Säuren sind anorganische Säuren wie Salzsäure, Bromwasserstoffsäure, Jodwasserstoffsäure, Schwefelsäure, Phosphorsäure, oder Salpetersäure oder organische Säuren wie Malonsäure, Oxalsäure, Gluconsäure, Camphersulfonsäure, Benzolsulfonsäure, Essigsäure, Propionsäure oder p-Toluolsulfonsäure.

Als physiologisch hydrolysierbare und akzeptable Derivate sind beispielsweise an der Hydroxygruppe veresterte Derivate geeignet, die unter physiologischen Bedingungen zu den freien Säuren hydrolysierbar sind, die ihrerseits wieder physiologisch akzeptierbar sind, d.h. bei den notwendigen Dosen nicht toxisch sind.

Die Verbindungen (I) weisen asymmetrische C-Atome auf und können daher als Enantiomere und Diastereomere auftreten. Die Erfindung umfaßt sowohl die reinen Isomeren als auch deren Gemische. Die Gemische von Diastereomeren können

nach gebräuchlichen Methoden, zum Beispiel durch selektive Kristallisation aus geeigneten Lösungsmitteln oder durch Chromatographie an Kieselgel oder Aluminiumoxid in die Komponenten aufgetrennt werden. Racemate können nach üblichen Methoden in die Enantiomeren aufgetrennt werden, so zum Beispiel durch Salzbildung mit einer optisch aktiven Säure, Trennung der diastereomeren Salze und Freisetzung der reinen Enantiomeren mittels einer Base.

Die Erfindung betrifft weiterhin ein Verfahren zur Herstellung von Verbindungen der Formel (I), dadurch gekennzeichnt, daß man eine Verbindung der Formel (II)

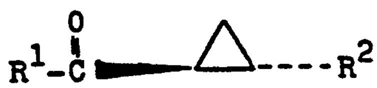

(II)

worin $R^1$ und $R^2$ die in Anspruch 1 angegebenen Bedeutungen haben, mit einem Schwefelylid der Formel (III),

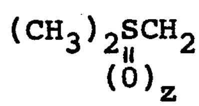

(III)

worin z 0 oder 1 bedeutet,
zu einer Verbindung der Formel (IV) umsetzt,

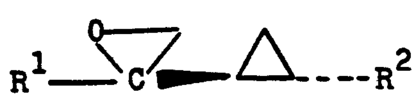

(IV)

worin $R^1$ und $R^2$ die oben angegebenen Bedeutungen haben, und diese Verbindung (IV) anschließend mit einer Verbindung der allgemeinen Formel (V) umsetzt,

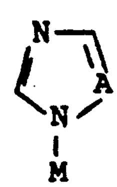

(V)

worin

A die in Anspruch 1 angegebenen Bedeutungen hat und

M für Wasserstoff, eine Trimethylsilylgruppe oder ein Metalläquivalent steht,

und eine so erhaltene Verbindung der Formel (I) in Form der freien Base oder eines Säureadditionssalzes oder eines physiologisch hydrolysierbaren und akzeptablen Derivats isoliert.

Zur Herstellung der Verbindungen der Formel (I) wird in einem ersten Reaktionsschritt zur Herstellung der Verbindungen der Formel (IV) ein Keton der Formel (II), in der $R^1$ und $R^2$ die oben angegebenen Bedeutungen haben, mit einem Schwefelylid der Formel (III) in einem inerten Lösungsmittel, vorzugsweise Dimethylsulfoxid, oder in Gemischen von Dimethylsulfoxid mit anderen inerten Lösungsmitteln, z.B. Tetrahydrofuran, umgesetzt. Dabei ist bei Verwendung eines Schwefelylids der Formel (III), für das z = 0 ist, ein Temperaturbereich zwischen -10° und 50°C, vorzugsweise zwischen 0° und 30°C zweckmäßig; bei Verwendung eines Schwefelylids der Formel (III), für das z = 1 ist, ein Temperaturbereich zwischen 0° und 80°, vorzugsweise zwischen 20° und 50°.

Die Herstellung der Schwefelylide (III) erfolgt nach in der Literatur beschriebenen Verfahren, die Herstellung der Cyclopropylketone (II) ebenfalls analog zu in der Literatur beschriebenen Verfahren.

Die Umwandlung der Zwischenprodukte der Formel (IV) in die Endprodukte der Formel (I) erfolgt in einem zweiten Reaktionsschritt durch Umsetzung mit einer Verbindung der Formel (V), in der A und M die oben angegebenen Bedeutungen haben, in einem inerten Lösungsmittel, z.B. N,N-Dimethylformamid, N,N-Dimethylacetamid, Dimethylsulfoxid, Acetonitril, Benzonitril, Sulfolan, N-Methylpyrrolidon, Dioxan oder Tetrahydrofuran, in einem Temperaturbereich zwischen 20° und 150°C.

Die Verbindung (V) kann dabei in Form eines Alkali- oder Erdalkalimetallsalzes, bevorzugt als Natriumsalz, oder in Form der freien Base in Gegenwart einer starken Base, umgesetzt werden. Geeignete Basen sind z.B. Alkali- oder Erdalkalimetallhydride, -amide oder alkoholate.

Der zweite Reaktionsschritt kann auch so durchgeführt werden, daß man die Verbindung der Formel (IV) mit Imidazol oder Triazol in äquivalenten Mengen, oder auch mit Imidazol oder Triazol im Überschuß, ohne Lösungsmittel in einem Temperaturbereich von 90 - 160°C zur Reaktion bringt.

Die Erfindung betrifft weiterhin ein Verfahren (b) zur Herstellung der Verbindungen der Formel (I), nach welchem

man eine Verbindung der Formel (II)

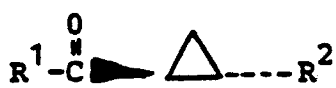

(II)

in der $R^1$ und $R^2$ wie oben definiert sind, mit einem Schwefelylid der Formel (III)

$$(CH_3)_2\underset{\underset{(O)_z}{\|}}{S}CH_2 \qquad \text{(III)}$$

in der z wie oben definiert ist, zu einer Verbindung der Formel (IV)

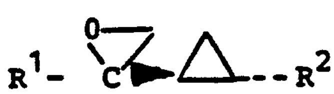

(IV)

umsetzt und diese anschließend ohne Aufarbeitung des Reaktionsgemisches und ohne Isolierung der Verbindung der Formel (IV) durch Zugabe einer Verbindung der Formel (V)

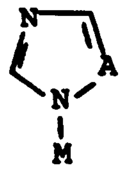

(V)

in welcher A und M wie oben definiert sind, zum Reaktionsgemisch zu einer Verbindung der Formel (I) umsetzt und diese in Form der freien Base oder eines Säureadditionssalzes oder eines physiologisch hydrolysierbaren und akzeptablen Derivats isoliert.

Die Umsetzung der Verbindungen der Formel (II) mit den Verbindungen der Formel (III) zu den Verbindungen der Formel (IV) und Umsetzung der letzteren mit solchen der Formel (V) zu den Endprodukten erfolgt dabei im Eintopfverfahren. Hierzu stellt man zunächst, wie im vorstehenden Verfahren beschrieben, eine Lösung einer Verbindung der Formel (IV) in einem der oben beschriebenen inerten Lösungsmittel her und gibt anschließend zu dieser Lösung eine Verbindung der Formel (V) und gegebenenfalls ein weiteres Äquivalent der oben genannten Basen zu und verfährt weiter wie im vorstehenden Verfahren beschrieben.

Die Erfindung betrifft weiterhin ein Verfahren c) zur Herstellung der Verbindungen der Formel (I), nach welchem man

eine Verbindung der Formel (VI),

$$R^1-\underset{\underset{O}{\|}}{C}-CH=CH-R^2 \qquad (VI),$$

worin $R^1$ und $R^2$ die oben angegebenen Bedeutungen haben, mit zwei Äquivalenten des Schwefelylids der Formel (IIIa)

$$(CH_3)_2\underset{\underset{O}{\|}}{S}CH_2 \qquad (III\ a)$$

zu einer Verbindung der Formel (IV)

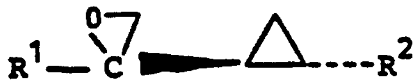 (IV)

umsetzt,
und anschließend die Verbindung der Formel (IV) ohne Isolierung der erhaltenen Verbindungen der Formel (IV) durch Zugabe einer Verbindung der Formel (V)

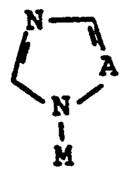 (V)

zum Reaktionsgemisch zur Verbindung der Formel (I) umsetzt und diese in Form der freien Base oder eines Säureadditionssalzes oder eines physiologisch hydrolysierbaren und akzeptablen Derivat isoliert, oder

ein Verfahren d), nach welchem man eine Verbindung der Formel (VI)

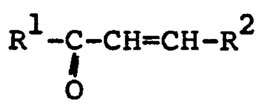 (VI)

in welcher $R^1$ und $R^2$ wie oben definiert sind, mit einem Äquivalent des Schwefelylids (III a)

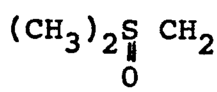 (III a)

zu einer Verbindung der Formel (II)

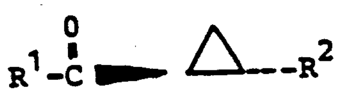 (II)

und diese anschließend ohne Aufarbeitung des Reaktionsgemisches und ohne Isolierung der Verbindung der Formel (II)

mit einem Äquivalent eines Schwefelylids der Formel (IIIb)

$(CH_3)_2SCH_2$ (III b)

zu einer Verbindung der Formel (IV) umsetzt und anschließend die Verbindung der Formel (IV) ohne Aufarbeitung des erhaltenen Reaktionsgemisches und ohne Isolierung der Verbindung der Formel (IV) durch Zugabe einer Verbindung der Formel (V)

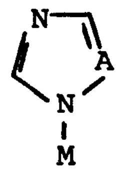

(V)

zum Reaktionsgemisch zu einer Verbindung der Formel (I) umsetzt und diese in Form der freien Base oder eines Säureadditionssalzes oder eines physiologisch hydrolylierbaren und akzeptablen Derivats isoliert.

Die Herstellung der Verbindungen (I) nach Variante c) erfolgt so, daß man zwei Äquivalente Dimethylsulfoxoniummethylid (III a) mit einem Äquivalent der Verbindung der Formel (VI) in einem inerten Lösungsmittel, vorzugsweise Dimethylsulfoxid, oder in Gemischen von Dimethylsulfoxid mit anderen inerten Lösungsmitteln, z.B. Tetrahydrofuran, bei einer Temperatur zwischen 20° und 80°C, vorzugsweise zwischen 20° und 50°C umsetzt, so die Verbindung der Formel (IV) erhält und anschließend zu dieser Lösung eine Verbindung der Formel (V), entweder in Form der oben erwähnten Metallsalze, vorzugsweise des Natriumsalzes, oder in Form der freien Base unter Zugabe eines Äquivalents der oben erwähnten starken Base, z.B. Natriumhydrid oder -amid, zugibt und weiter wie in den vorstehenden Verfahren verfährt.

Die Ausgangsprodukte der Formel (VI) sind bekannt oder können nach bekannten Verfahren enthalten werden.

Die Herstellung der Verbindungen (I) nach Variante d) erfolgt so, daß man unter den unter c) beschriebenen Reaktionsbedingungen ein Äquivalent Dimethylsulfoxoniummethylid (III a) mit einem Äquivalent der Verbindung (VI) zu einer Verbindung der Formel (II) umsetzt, anschließend zur Reaktionslösung bei einer Temperatur zwischen -10°C und 50°C, vorzugsweise 0° - 30°C, eine Lösung von Dimethylsulfoniummethylid (III b) in einem inerten Lösungsmittel, vorzugsweise Dimethylsulfoxid, zugibt und zu dieser Lösung, analog wie unter Variante c) beschrieben, eine Verbindung der Formel (V) zugibt und weiter wie unter Variante d) beschrieben verfährt.

Die Verbindungen der Formel I, ihre Säureadditionssalze und ihre physiologisch hydrolysierbaren Derivate sind wertvolle Arzneimittel. Sie wirken insbesondere antimikrobiell und eignen sich zur Vorbeugung und Behandlung von Pilzinfektionen beim Menschen und bei verschiedenen Säugetierarten.

Die neuen Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen grampositive und gramnegative Bakterien.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Ebenso besteht ein sehr guter Effekt gegen verschiedene Erreger der Hautmykosen (z.B.

Trichophyton mentagrophytes) am Meerschweinchen nach oraler, parenteraler oder lokaler Anwendung.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiete in der Tiermedizin können beispielsweise aufgeführt werden:

Alle Dermatomykosen und Systemmykosen, insbesondere solche, die durch die oben genannten Erreger hervorgerufen werden.

Zur vorliegenden Erfindung gehören phamazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,05 bis etwa 200, vorzugsweise 0,1 bis 100, insbesondere 0,5 bis 10 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8,, vorzugsweise in 1 bis 3 Einzelsdosen verabreicht.

Es kann jedoch erforderlich sein, von den genannten Dosierungen abzuweichen und zwar in Abhängigkeit von der

Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die neuen Verbindungen der allgemeinen Formel (I) sind auch als Biozide wirksam, vorzugsweise die Triazolverbindungen, in denen A Stickstoff bedeutet. Sie zeichnen sich insbesondere durch ihre fungizide Wirksamkeit bei phytopathogenen Pilzen aus. Selbst bereits in das pflanzliche Gewebe eingedrungene pilzliche Krankheitserreger lassen sich erfolgreich bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der neuen Verbindungen erfaßt eine Vielzahl verschiedener phytopathogener Pilze, wie z.B. Piricularia oryzae, Plasmopara viticola, verschiedene Rostarten, vor allem aber Venturia inaequalis, Cercospora beticola und echte Mehltaupilze im Obst-, Gemüse-, Getreide- und Zierpflanzenbau.

Die neuen Verbindungen der allgemeinen Formel (I) eignen sich ferner für den Einsatz in technischen Bereichen, beispielsweise als Holzschutzmittel, als Konservierungsmittel in Anstrichfarben, in Kühlschmiermitteln für die Metallbearbeitung oder als Konservierungsmittel in Bohr- und Schneidölen.

Die neuen Verbindungen können als Spritzpulver, emulgierbare Konzentrate, versprühbare Lösungen, Stäubemittel, Beizmittel, Dispersionen, Granulate oder Mikrogranulate in den üblichen Zubereitungen angewendet werden.

Unter Spritzpulvern werden in Wasser gleichmäßig dispergierbare Präparate verstanden, die neben dem Wirkstoff außer gegebenenfalls einem Verdünnungs- oder Inertstoff noch Netzmittel, z.B. polyoxethylierte Alkylphenole, polyoxethylierte Fettalkohole, Alkyl- oder Alkylphenylsulfonate und Dispergiermittel z.B. ligninsulfonsaures Natrium, 2,2'-dinapthylmethan-6,6'-di-sulfonsaures Natrium, dibutylnaphthalin-sulfonsaures Natrium oder auch oleoylmethyltaurinsaures Natrium enthalten. Ihre Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze, wie Ca-dodecylbenzolsulfonat, oder nichtionische Emulgatoren wie Fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel werden durch Vermahlen des Wirkstoffes mit fein verteilten, festen Stoffen, z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinit oder von granuliertem Inertmaterial. Auch können geeignete Wirkstoffe in der für die Herstellung von Düngemittelgranulaten üblichen Weise - gewünschtenfalls in Mischung mit Düngemitteln, granuliert werden.

In Spritzpulvern beträgt die Wirkstoffkonzentration z.B. etwa 10-90 Gew.-%, der Rest zu 100 Gew.-% besteht aus üblichen Formulierungsbestandteilen. Bei emulgierbaren Konzentraten kann die Wirkstoffkonzentration etwa 10-80 Gew.-% an Wirkstoff, bei versprühbaren Lösungen etwa 1-20 Gew.-% betragen. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Auch Mischungen oder Mischformulierungen mit anderen Wirkstoffen, wie z.B. Insektiziden, Akariziden, Herbiziden,

Düngemitteln, Wachstumsregulatoren oder weiteren Fungiziden sind gegebenenfalls möglich, wobei u.U. auch synergistische Wirkungssteigerungen erzielt werden können.

Im folgenden seien einige Formulierungsbeispiele angeführt:

Ein Stäubemittel wird erhalten, indem man 10 Gewichtsteile Wirkstoff und 90 Gewichtsteile Talkum als Inertstoff mischt und in einer Schlagmühle zerkleinert.

Ein in Wasser leicht dispegierbares, benetzbares Pulver wird erhalten, indem man 25 Gewichtsteile Wirkstoff, 65 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

Ein in Wasser leicht dispergierbares Dispersionskonzentrat stellt man her, indem man 20 Gewichtsteile Wirkstoff mit 6 Gewichtsteilen Alkylphenolpolyglykolether (Triton X 207), 3 Gewichtsteilen Isotridecanolpolyglykolether (8 AeO) und 71 Gewichtsteilen paraffinischem Mineralöl (Siedebereich z.B. ca. 255 bis über 377°C) mischt und in einer Reibkugelmühle auf eine Feinheit von unter 5 Mikron vermahlt.

Ein emulgierbaren Konzentrat läßt sich herstellen aus 15 Gewichtsteilen Wirkstoff, 75 Gewichtsteilen Cyclohexanon als Lösungsmittel und 10 Gewichtsteilen oxethyliertem Nonylphenol (10 AeO) als Emulgator.

Die nachfolgenden Beispiele dienen zur näheren Erläuterung der Erfindung, ohne dieselbe einzuschränken.

Beispiel 1

E-1-[1-(4-Chlorphenyl)-1-hydroxy-2-(1,2,4-triazol-1-yl)-1-ethyl]-2-(1-naphtyl)-cyclopropan

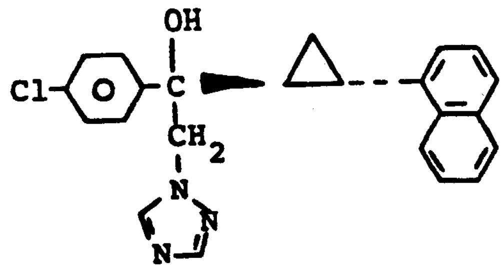

Zu einer Suspension von 1,8 g Natriumhydrid (80 %ige Dispersion in Öl), in 75 ml trockenem Dimethylsulfoxid wurden portionsweise unter Kühlung 11,0 g Trimethylsulfoxoniumjodid gegeben. Man rührte bis zum Ende der Gasentwicklung und gab anschließend eine Lösung von 19,5 g E-1-(4-Chlorbenzoyl)-2-(1-naphthyl)-cyclopropan in 50 ml Dimethylsulfoxid/Tetrahydrofuran 1:1 zu. Man rührte 3 Stunden bei Zimmertemperatur und 2 Stunden bei 50°C. Nach Abkühlen gab man 4,6 g Triazol-Natriumsalz zu, rührte weitere 3 Stunden bei Zimmertemperatur und 1 Stunde bei 50°C. Zur Aufarbeitung wurde auf Wasser gegossen, mit Methylenchlorid ausgeschüttelt, die organische Phase getrocknet und eingeengt. Zur Reinigung und Trennung der Diastereomeren wurde an Kieselgel mit Ethylacetat chromatographiert. Die Verbindungen wurden als Öle erhalten, die nach Verreiben mit Ether kristallisierten. Das als erstes von der Säule eluierte Isomere hat einen Schmelzpunkt von 174 - 176°C, das zweite schmilzt bei 161 - 164°C. Gesamtausbeute 7,8 g (40 % d.Th.)

Herstellung des Ausgangsprodukts

E-1-(4-Chlorbenzoyl)-2-(2-naphthyl)-cyclopropan.

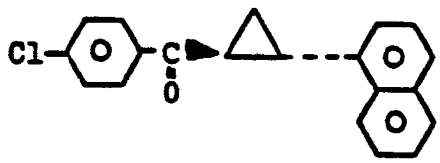

Zu einer Suspension von 3,6 g Natriumhydrid (80 %ige Dispersion in Öl) in 150 ml trockenem Dimethylsulfoxid gab man portionsweise untere Kühlung 24,0 g Trimethylsulfoxoniumjodid und rührte bis zum Ende der Gasentwicklung Nun gab man eine Lösung von 29,3 g 3-(4-Chlorphenyl)-1-(1-naphtyl)-1-propen-3-on (durch Aldolkondensation aus 1-Naphtaldehyd und 4-Chloracetophenon erhalten) in 150 ml Dimethylsulfoxid / Tetrahydrofuran 1:1 zu, rührte 3 Stunden bei Zimmertemperatur und eine Stunde bei 50°C. Zur Aufarbeitung wurde auf Wasser gegossen, mit Methylenchlorid ausgeschüttelt, die organische Phase getrocknet und eingeengt. Zur Reinigung aus Ethanol umkristallisiert. Man erhielt 22,4 g farblose Substanz (73 % d.Th.) vom Fp. 94-95°C.

Beispiel 2

E-1-[1-(2,4-Dichlorphenyl)-1-hydroxy-2-(1H-imidazol-1-yl)-1-ethyl]-2-(2,4-dichlorphenyl)-cyclopropan

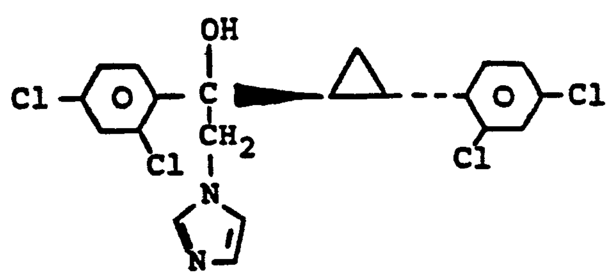

Zu einer Suspension von 10,8 g Natriumhydrid (80 %ige Dispersion in Öl) (0,36 mol) in 400 ml trockenem Dimethylsulfoxid gab man unter Kühlung portionsweise 80,0 g Trimethylsulfoxoniumjodid (0,36 mol) und rührte bis zum Ende der Gasentwicklung. Nun gab man eine Lösung von 51,9 g 1,3-Bis-(2,4-dichlorphenyl)-1-propen-3-on (0,15 mol) (hergestellt durch Aldolkondensation aus 2,4-Dichlorbenzaldehyd und 2,4-Dichloracetophenon) in 250 ml Dimethylsulfoxid / Tetrahydrofuran 1:1 zu, rührte 3 Stunden bei Zimmertemperatur und 1 Stunde bei 50°C. Bei dieser Temperatur gab man 13,5 g Imidazol-Natriumsalz zu, rührte weitere 2 Stunden bei 50°C und 6 Stunden bei 80°C. Zur Aufarbeitung wurde auf Wasser gegossen, mit Methylenchlorid ausgeschüttelt, die organische Phase getrocknet und eingeengt. Zur Trennung der Diastereomeren wurde das Rohprodukt an Kieselgel mit Ethylacetat / Methanol 9:1 chromatographiert. Beide Verbindungen wurden als Öle erhalten, die beim Verreiben mit Ether kristallisierten. Das zuerst eluierte Isomere (15,6 g, 23,5 % d.Th.) schmilzt bei 183 - 184°C, das zuletzt eluierte (27,0 g, 40.7 % d.Th.) bei 165 - 167°C.

Beispiel 3

E-1-[1-(2,4-Dichlorphenyl)-1-hydroxy-2-(1,2,4-triazol-1-yl)-1-ethyl]-2-(2,4-dichlorphenyl)-cyclopropan

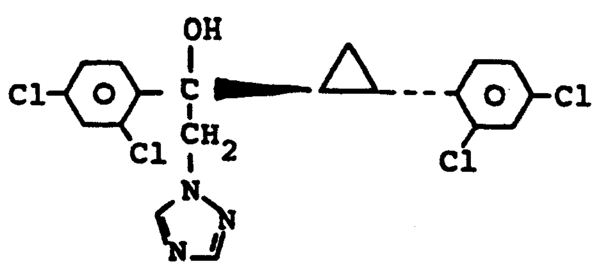

Völlig analog Beispiel 2, aber unter Verwendung von 13,7 g 1,2,4-Triazol-Natriumsalz wurde ein Gemisch zweier Diastereomerer erhalten, die durch Chromatographie an Kieselgel mit Ethylacetat getrennt wurden. Das zuerst eluierte

Isomere (4,9 g, 7,9 % d.Th.) schmilzt bei 166 - 167°C das zuletzt eluierte (8,6 g, 12,9 % d.Th.) bei 83 - 85°C.

Analog Beispiel 2) bzw. 3) können auch die in Tabelle 1 aufgeführten Verbindungen der Formel (I) erhalten werden.

Die unter einer Ziffer jeweils mit a und b gekennzeichneten Verbindungen sind Diastereomere. Dabei ist das mit a bezeichnete Isomere dasjenige Diastereomere, das im DC jeweils den größeren $R_f$-Wert aufweist.

| Beisp.-Nr. | $R^1$ | $R^2$ | A | Fp. [°C] |
|---|---|---|---|---|
| 1 a | $4-C_6H_4Cl$ | 1-Naphthyl | N | 174-176 |
| 1 b | " | | | 161-164 |
| 2 a | $2.4-C_6H_3Cl_2$ | $2.4-C_6H_3Cl_2$ | CH | 183-184 |
| 2 b | | | | 165-167 |
| 3 a | " | " | N | 166-167 |
| 3 b | | | | 83- 85 |
| 4 a | $4-C_6H_4Cl$ | $4-C_6H_4Cl$ | CH | 182-185 |
| 4 b | | | | Harz |
| 5 a | " | $4-C_6H_4F$ | N | 115-117 |
| 5 b | | | | 132-134 |
| 6 a | " | " | CH | 157-158 |
| 6 b | | | | 142-144 |
| 7 a | " | $4-C_6H_4CF_3$ | N | |
| 7 b | | | | |
| 8 a | " | " | CH | |
| 8 b | | | | |
| 9 a | " | $4-C_6H_4OCH_3$ | N | 117-118 |
| 9 b | | | | 132-133 |
| 10 a | " | " | CH | 161-162 |
| 10 b | | | | 139-140 |
| 11 a | " | $4-C_6H_4CH_3$ | N | |
| 11 b | | | | |
| 12 a | " | " | CH | |
| 12 b | | | | |

| Beisp.-Nr. | $R^1$ | $R^2$ | A | Fp. [°C] |
|---|---|---|---|---|
| 13 a | 4-$C_6H_4Cl$ | 4-Biphenylyl | CH | |
| 13 b | | | | |
| 14 a | " | 4-Phenoxyphenyl | CH | 194-196 |
| 14 b | | | | 141-143 |
| 15 a | " | $C_6H_5$ | N | 90 - 92 |
| 15 b | | | | Harz |
| 16 a | " | 1-Naphthyl | CH | Harz |
| 16 b | | | | 201 - 202 |
| 17 a | " | 3,4-$C_6H_3Cl_2$ | N | |
| 17 b | | | | |
| 18 a | " | " | CH | |
| 18 b | | | | |
| 19 a | " | 2,4-$C_6H_3Cl_2$ | N | Harz |
| 19 b | | | | Harz |
| 20 a | " | " | CH | 145 - 147 |
| 20 b | | | | 145 - 147 |
| 21 a | 4-$C_6H_4F$ | $C_6H_5$ | N | 100 - 102 |
| 21 b | | | | 122 - 123 |
| 22 a | " | " | CH | 168 - 169 |
| 22 b | | | | 164 - 165 |
| 23 a | " | 4-$C_6H_4Cl$ | N | 163 - 165 |
| 23 b | | | | 122 - 124 |
| 24 a | " | " | CH | 178 - 180 |
| 24 b | | | | 168 - 170 |

| Beisp.-Nr. | $R^1$ | $R^2$ | A | Fp. [°C] |
|---|---|---|---|---|
| 25 a | $4-C_6H_4F$ | $4-C_6H_4F$ | N | 141 - 142 |
| 25 b | | | | 139 - 140 |
| 26 a | " | " | CH | 177 - 178 |
| 26 b | | | | 168 - 170 |
| 27 a | " | $2.4-C_6H_3Cl_2$ | N | 134 - 135 |
| 27 b | | | | 136 - 137 |
| 28 a | " | " | CH | 177 - 179 |
| 28 b | | | | 205 - 206 |
| 29 a | $2,4-C_6H_3Cl_2$ | $4-C_6H_4Cl$ | N | 164 - 166 |
| 29 b | | | | 143 - 145 |
| 30 a | " | " | CH | 199 - 203 |
| 30 b | | | | 194 - 197 |
| 31 a | " | $4-C_6H_4-F$ | N | 167 - 168 |
| 31 b | | | | 158 - 159 |
| 32 a | " | " | CH | 174 - 175 |
| 32 b | | | | 170 - 172 |
| 33 a | $4-C_6H_4Cl$ | $C_6H_5$ | CH | 141 - 143 |
| 33 b | | | | 152 - 154 |
| 34 a | " | $4-C_6H_4Cl$ | N | 106 - 108 |
| 34 b | | | | 126 - 127 |
| 35 a | $2.4-C_6H_3Cl_2$ | 2-Thienyl | N | |
| 35 b | | | | |
| 36 a | " | " | CH | 188 - 189 |
| 36 b | | | | Harz |

| Beisp.-Nr. | $R^1$ | $R^2$ | A | Fp. [°C] |
|---|---|---|---|---|
| 37 a | 2.4-$C_6H_3Cl_2$ | 4-$C_6H_4CF_3$ | N | 186 - 187 |
| 37 b | | | | 84 - 86 |
| 38 a | 4-$C_6H_4Cl$ | 2.4-$C_6H_3F_2$ | N | 102 - 103 |
| 38 b | | | | 139 - 140 |
| 39 a | 4-$C_6H_4F$ | " | N | 124 - 126 |
| 39 b | | | | 149 - 151 |
| 40 a | 2.4-$C_6H_3F_2$ | 4-$C_6H_4Cl$ | N | 121 - 123 |
| 40 b | | | | 128 - 129 |
| 41 a | " | 4-$C_6H_4F$ | N | |
| 41 b | | | | |
| 42 a | 4-$C_6H_4F$ | 2-$C_6H_4Cl$ | N | |
| 42 b | | | | |
| 43 a | 2-$C_6H_4Cl$ | 4-$C_6H_4F$ | N | |
| 43 b | | | | |
| 44 a | 2-Thienyl | 4-$C_6H_4Cl$ | CH | |
| 44 b | | | | |
| 45 a | 4-$C_6H_4Cl$ | 3,4-$C_6H_4F_2$ | N | |
| 45 b | | | | |
| 46 a | " | " | CH | |
| 46 b | | | | |
| 47 a | 4-$C_6H_4F$ | " | N | |
| 47 b | | | | |
| 48 a | " | " | CH | |
| 48 b | | | | |

0 212 605

| Beisp.-Nr. | $R^1$ | $R^2$ | A | Fp. [°C] |
|---|---|---|---|---|
| 49 a | 4-$C_6H_4Cl$ | 4-Phenoxyphenyl | N | 88 - 90 |
| 49 b | | " | " | 126 - 128 |
| 50 a | " | 2,4-$C_6H_3F_2$ | CH | 134 - 136 |
| 50 b | " | " | " | 145 - 146 |
| 51 a | 4-$C_6H_4F$ | " | " | 126 - 128 |
| 51 b | | " | " | 148 - 150 |
| 52 a | 2,4-$C_6H_3Cl_2$ | 3,4-$C_6H_3F_2$ | " | 175 - 176 |
| 52 b | " | " | " | 181 - 183 |
| 53 a | " | " | N | 181 - 182 |
| 53 b | " | " | " | 134 - 135 |
| 54 a | " | 5-Cl-Thien-2-yl | CH | 175 - 176 |
| 54 b | " | " | " | 172 - 173 |
| 55 a | " | " | N | 162 - 163 |
| 55 b | " | " | " | 165 - 166 |
| 56 a | 2,4-$C_6H_3F_2$ | 2,4-$C_6H_3Cl_2$ | " | 128 - 130 |
| 56 b | " | " | " | 105 - 107 |
| 57 a | " | " | CH | 116 - 118 |
| 57 b | " | " | " | 156 - 157 |
| 58 a | " | 2,4-$C_6H_3F_2$ | " | 156 - 157 |
| 58 b | " | " | " | 164 - 166 |
| 59 a | " | " | N | 126 - 128 |
| 59 b | " | " | " | 126 - 128 |

| Beisp.-Nr. | $R^1$ | $R^2$ | A | Fp. [°C] |
|---|---|---|---|---|
| 60 a | $2,4-C_6H_3F_2$ | $4-C_6H_4Cl$ | CH | 156 - 157 |
| 60 b | " | " | " | 138 - 139 |
| 61 a | $4-Cl,2-F-C_6H_3$ | " | " | 114 - 116 |
| 61 b | " | " | " | 159 - 161 |
| 62 a | " | " | N | 123 - 124 |
| 62 b | " | " | N | 144 - 145 |
| 63 a | $4-F,2-Cl-C_6H_3$ | " | CH | 168 - 170 |
| 63 b | " | " | " | |
| 64 a | 2,4-Dichlorthien-5-yl | " | " | 213 - 215 |
| 64 b | " | " | " | |
| 65 a | " | " | N | 123 - 127 |
| 65 b | " | " | N | Harz |

Als Beispiel für die hohe orale und parenterale in-vivo-Wirksamkeit der erfindungsgemäßen Verbindungen werden Behandlungsergebnisse an experimentell mit Candida albicans infizierten Labortieren angeführt.

Zur Feststellung der oralen und parenteralen Wirksamkeit wurden Gruppen von je 5 18-20 g schweren Mäusen (Stamm HOE: NMRKF; SPF 71) mit 2 . $10^6$ Keimen/Tier infiziert.

Die Behandlung der Tiere erfolgte oral bzw. subcutan in 8 gleichen Einzeldosen zu je 30 mg/kg bzw. 10 mg/kg Körpergewicht (-24/-18/-2h/+2/24/30/48/54h).

Neben der mit den erfindungsgemäßen Substanzen I behandelten Gruppe von 5 Tieren wurde zum Vergleich eine Gruppe von ebenfalls 5 Tieren mit der Referenzsubstanz Ketoconazol behandelt. Eine kontrollgruppe von 10 Tieren blieb nach der Infektion unbehandelt.

Wie aus Tabelle 2 hervorgeht, fand sich bei den erfindungsgemäßen Verbindungen eine bis um das Doppelte verlängerte Überlebenszeit der Tiere nach der Infektion, verglichen mit dem derzeitigen Standardpräparat Ketoconazol.

Tabelle 2

| Dosis | Präparat Beispiel Nr. | Anzahl/Tiere | Überlebenszeiten Tage nach Infektion | $\bar{d}$ Tage | Überlebenszeit in % (Standardpräp.= 100%) |
|---|---|---|---|---|---|
| Oral | 34 b | 5 | 10 13 14 14 14 | 13,0 | 162,5 |
| 8x30 mg/kg | 27 b | 5 | 10 10 10 14 14 | 11,6 | 145,0 |
| | 29 a | 5 | 9 9 10 10 11 | 9,8 | 122,5 |
| | Ketoconazol | 5 | 6 7 9 9 9 | 8,0 | 100,0 |
| Oral | 34 b | 5 | 10 11 12 12 13 | 11,6 | 207,1 |
| 8x10 mg/kg | 27 b | 5 | 8 8 10 10 10 | 9,2 | 164,3 |
| | 29 a | 5 | 5 6 6 7 10 | 6,8 | 121,4 |
| | Ketoconazol | 5 | 5 5 5 6 7 | 5,6 | 100,0 |
| Subcutan | 34 b | 5 | 3 4 10 11 12 | 8,0 | 100,0 |
| 8x30 mg/kg | 27 b | 5 | 11 12 12 14 14 | 12,6 | 157,5 |
| | 29 a | 5 | 10 11 12 12 12 | 11,4 | 142,5 |
| | Ketoconazol | 5 | 8 8 8 8 8 | 8,0 | 100,0 |
| Subcutan | 34 b | 5 | 10 10 11 12 12 | 11,0 | 171,9 |
| 8x10 mg/kg | 27 b | 5 | 10 10 10 11 11 | 10,4 | 162,5 |
| | 29 a | 5 | 6 6 7 7 10 | 7,2 | 112,5 |
| | Ketoconazol | 5 | 5 5 5 8 9 | 6,4 | 100,0 |
| infizierte Tiere | - | 10 | 1 1 1 1 2 | 1,8 | 25,7 |
| unbehandelt | | | 2 2 2 3 3 | | |

PATENTANSPRÜCHE:

1. Azolverbindung der Formel I

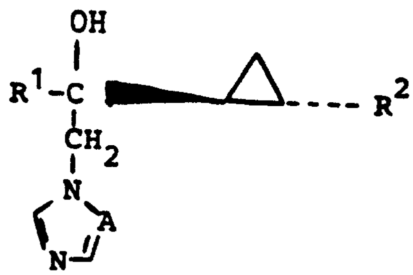

(I)

worin
A CH oder N bedeutet und
$R^1$ und $R^2$ gleich oder verschieden sind und jeweils Phenyl, Biphenyl, Phenoxyphenyl, Benzylphenyl, Benzyloxyphenyl, Naphthyl, Thienyl, Furyl oder Pyridyl bedeuten, wobei die Ringe unsubstituiert oder durch einen, zwei oder drei Substituenten substituiert sind, die gleich oder verschieden sind und jeweils Fluor, Chlor, Brom, Jod $(C_1-C_8)$-Alkyl, unverzweigt oder verzweigt, unsubstituiert oder durch 1 - 9 Fluor- oder Chloratome substituiert, $(C_3-C_6)$-Cycloalkyl, $(C_3-C_6)$-Cycloalkyl$(C_1-C_4)$-Alkyl, $(C_1-C_8)$-Alkoxy, $(C_1-C_8)$-Alkylthio oder Nitro bedeuten,
sowie deren pharmazeutisch akzeptable Salze und Ester und ihre Stereoisomeren.

2. Verbindung der Formel I gemäß Anspruch 1, worin $R^1$ und $R^2$ jeweils gleich oder verschieden sind und unsubstituiertes Biphenyl, Phenoxyphenyl, Benzylphenyl, Benzyloxyphenyl, Naphthyl, Thienyl, Furyl oder Pyridyl oder eine unsubstituierte oder mit einem oder zwei Substituenten versehene Phenylgruppe bedeuten und diese Substituenten jeweils Fluor, Chlor, Brom, $(C_1-C_4)$-Alkyl, Trifluormethyl, $(C_1-C_4)$-Alkoxy, $(C_1-C_4)$-Alkylthio oder Nitro bedeuten.

3. Verbindung gemäß mindestens einem der Ansprüche 1 und 2 worin $R^1$ und $R^2$ die Phenylgruppe bedeutet, welche unsubstituiert oder durch einen oder zwei Substituenten substituiert ist, die Fluor, Chlor, Brom oder die Trifluormethylgruppe bedeuten.

4. Verbindung gemäß mindestens einem der Ansprüche 1 bis 3, worin die Substituenten von $R^1$ und $R^2$ jeweils Fluor oder Chlor bedeuten.

5. Verfahren zur Herstellung von Verbindungen der Formel I nach Anspruch 1, dadurch gekennzeichnet, daß man

a) eine Verbindung der Formel (II),

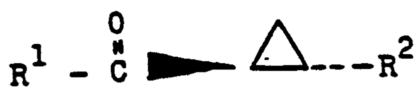

(II)

worin $R^1$ und $R^2$ die in Anspruch 1 angegebenen Bedeutungen haben, mit einem Schwefelylid der Formel (III),

$$(CH_3)_2\underset{(O)_z}{SCH_2} \qquad (III)$$

worin z 0 oder 1 bedeutet,
zu einer Verbindung der Formel (IV) umsetzt,

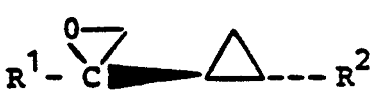

(IV)

worin $R^1$ und $R^2$ die oben angegebenen Bedeutungen haben, und diese Verbindung (IV) anschließend mit einer Verbindung der allgemeinen Formel (V) umsetzt,

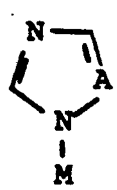

(V)

worin A die in Anspruch 1 angegebenen Bedeutungen hat und M für Wasserstoff, eine Trimethylsilylgruppe oder ein Metalläquivalent steht, und eine so erhaltene Verbindung der Formel (I) in Form der freien Base oder eines Säureadditionssalzes oder eines physiologisch hydrolysierbaren und akzeptablen Derivats isoliert, oder

b) eine Verbindung der Formel (II)

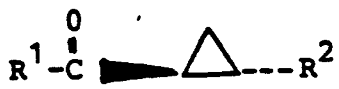

(II)

mit einem Schwefelylid der Formel (III)

$$(CH_3)_2\underset{(O)_z}{\overset{}{S}}CH_2 \qquad (III)$$

zu einer Verbindung der Formel (IV)

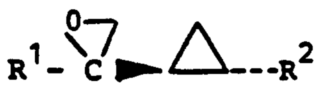

(IV)

umsetzt und diese anschließend ohne Isolierung der Verbindung der Formel (IV) durch Zugabe einer Verbindung der Formel (V)

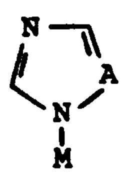

(V)

zum Reaktionsgemisch zu einer Verbindung der Formel (I) umsetzt und diese in Form der freien Base oder eines Säureadditionssalzes oder eines physiologisch hydrolysierbaren und akzeptablen Derivats isoliert, oder

c) eine Verbindung der Formel (VI)

$$R^1-\underset{\overset{\|}{O}}{C}-CH=CH-R^2 \qquad (VI)$$

worin $R^1$ und $R^2$ die in Anspruch 1 angegebenen Bedeutungen haben, mit zwei Äquivalenten des Schwefelylids der Formel (III a)

$$(CH_3)_2\underset{\overset{\|}{O}}{S}CH_2 \qquad (III\ a)$$

zu einer Verbindung der Formel (IV)

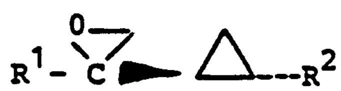

(IV)

umsetzt und anschließend die Verbindung der Formel (IV) ohne Isolierung der erhaltenen Verbindung der Formel (IV) durch Zugabe einer Verbindung der Formel (V)

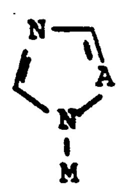

zum Reaktionsgemisch zur Verbindung der Formel (I) umsetzt und diese in Form der freien Base oder eines Säureadditionssalzes oder eines physiologisch hydrolysierbaren und akzeptablen Derivat isoliert, oder

d) eine Verbindung der Formel (VI) mit einem Äquivalent des Schwefelylids (III a) zu einer Verbindung der Formel (II)

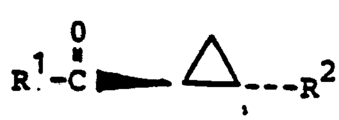

(II)

umsetzt und diese anschließend ohne Isolierung der Verbindung der Formel (II) mit einem Äquivalent des Schwefelylids der Formel (III b)

$(CH_3)_2SCH_2$ (III b)

zu einer Verbindung der Formel (IV)

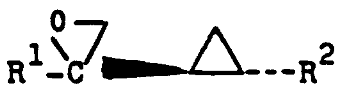 (IV)

umsetzt

und anschließend die Verbindung der Formel (IV) ohne Isolierung der erhaltenen Verbindung der Formel (IV) durch Zugabe einer Verbindung der Formel (V)

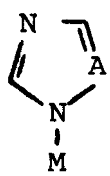

zum Reaktionsgemisch zur Verbindung der Formel (I) umsetzt und diese in Form der freien Base oder eines Säureadditionssalzes oder eines physiologisch hydrolysierbaren und akzeptablen Derivat isoliert.

6. Arzneimittel, dadurch gekennzeichnet, daß es mindestens eine Verbindung I gemäß Anspruch 1 enthält oder aus ihr besteht.

7. Verfahren zur Herstellung einer pharmazeutischen Zubereitung, dadurch gekennzeichnet, daß man eine Verbindung der Formel (I) oder eines ihrer Salze, gegebenenfalls zusammen mit einem üblichen pharmazeutischen Trägerstoff und/oder Hilfsstoff, in eine für pharmazeutische Zwecke geeignete Darreichungsform bringt.

8. Verwendung einer Verbindung I nach Anspruch 1 zur Bekämpfung von Mykosen, Protozoen und grampositiver und gramnegativer Bakterien.

9. Schädlingsbekämpfungsmittel, gekennzeichnet durch einen Gehalt an einer Verbindung der Formel (I) nach Anspruch 1.

10. Verfahren zur Bekämpfung oder Verhinderung eines Befalls von Kulturpflanzen durch phytopathogene Pilze, dadurch gekennzeichnet, daß man eine Verbindung der Formel (I) gemäß Anspruch 1 auf die Pflanze bzw. auf durch Pilzbefall bedrohte Flächen, Pflanzen oder Saatgüter einwirken läßt.

11. Verfahren zur Herstellung eines fungiziden Mittels gemäß Anspruch 9 , dadurch gekennzeichnet, daß man eine Verbindung der Formel (I) gemäß Anspruch 1 mit festen oder flüssigen Trägerstoffen sowie gegebenenfalls mit einem oder mehreren oberflächenaktiven Mitteln mischt.

12. Verwendung einer Verbindung I nach Anspruch 1 zur Herstellung eines Medikaments.

Patentansprüche Österreich:

1. Verfahren zur Herstellung von Verbindungen der Formel I

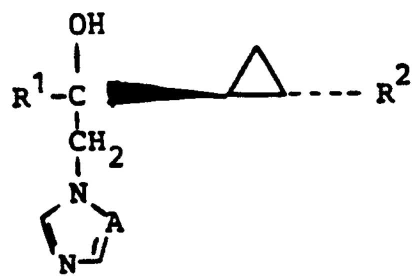

(I)

worin

A CH oder N bedeutet und

$R^1$ und $R^2$ gleich oder verschieden sind und jeweils Phenyl, Biphenyl, Phenoxyphenyl, Benzylphenyl, Benzyloxyphenyl, Naphthyl, Thienyl, Furyl oder Pyridyl bedeuten, wobei die Ringe unsubstituiert oder durch einen, zwei oder drei Substituenten substituiert sind, die gleich oder verschieden sind und jeweils Fluor, Chlor, Brom, Jod $(C_1-C_8)$-Alkyl, unverzweigt oder verzweigt, unsubstituiert oder durch 1 - 9 Fluor- oder Chloratome substituiert, $(C_3-C_6)$-Cycloalkyl, $(C_3-C_6)$-Cycloalkyl$(C_1-C_4)$-Alkyl, $(C_1-C_8)$-Alkoxy, $(C_1-C_8)$-Alkylthio oder Nitro bedeuten,

sowie deren pharmazeutisch akzeptable Salze und Ester und ihre Stereoisomeren, dadurch gekennzeichnet, daß man

a) eine Verbindung der Formel (II)

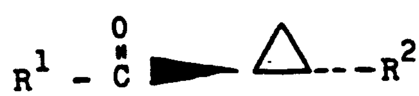

(II)

worin $R^1$ und $R^2$ die angegebenen Bedeutungen haben, mit

einem Schwefelylid der Formel (III),

$$(CH_3)_2\underset{(O)_z}{\overset{}{S}}CH_2 \qquad (III)$$

worin z 0 oder 1 bedeutet,
zu einer Verbindung der Formel (IV) umsetzt,

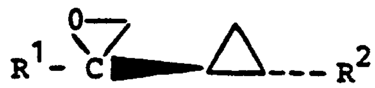

(IV)

worin $R^1$ und $R^2$ die oben angegebenen Bedeutungen haben, und diese Verbindung (IV) anschließend mit einer Verbindung der allgemeinen Formel (V) umsetzt,

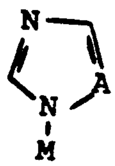

(V)

worin A die angegebenen Bedeutungen hat und M für Wasserstoff, eine Trimethylsilylgruppe oder ein Metalläquivalent steht,
und eine so erhaltene Verbindung der Formel (I) in Form der freien Base oder eines Säureadditionssalzes oder eines physiologisch hydrolysierbaren und akzeptablen Derivats isoliert, oder

b) eine Verbindung der Formel (II)

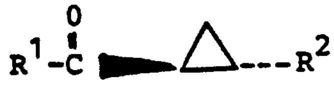

(II)

mit einem Schwefelylid der Formel (III)

$$(CH_3)_2\underset{(O)_z}{\overset{}{S}}CH_2 \qquad (III)$$

zu einer Verbindung der Formel (IV)

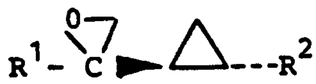

(IV)

umsetzt und diese anschließend ohne Isolierung der Verbindung der Formel (IV) durch Zugabe einer Verbindung der Formel (V)

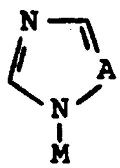

(V)

zum Reaktionsgemisch zu einer Verbindung der Formel (I) umsetzt und diese in Form der freien Base oder eines Säureadditionssalzes oder eines physiologisch hydrolysierbaren und akzeptablen Derivats isoliert; oder

c) eine Verbindung der Formel (VI)

$$R^1-\underset{\underset{O}{\parallel}}{C}-CH=CH-R^2 \qquad (VI)$$

worin $R^1$ und $R^2$ die angegebenen Bedeutungen haben, mit zwei Äquivalenten des Schwefelylids der Formel (III a)

$$(CH_3)_2\underset{\underset{O}{\parallel}}{S}CH_2 \qquad (III\ a)$$

zu einer Verbindung der Formel (IV)

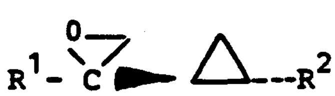

(IV) ;

umsetzt und anschließend die Verbindung der Formel (IV)

ohne Isolierung der erhaltenen Verbindung der Formel (IV) durch Zugabe einer Verbindung der Formel (V)

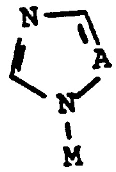

zum Reaktionsgemisch zur Verbindung der Formel (I) umsetzt und diese in Form der freien Base oder eines Säureadditionssalzes oder eines physiologisch hydrolysierbaren und akzeptablen Derivat isoliert, oder

d) eine Verbindung der Formel (VI) mit einem Äquivalent des Schwefelylids (III a) zu einer Verbindung der Formel (II)

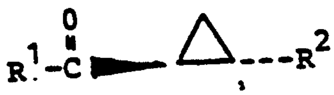

(II)

umsetzt und diese anschließend ohne Isolierung der Verbindung der Formel (II) mit einem Äquivalent des Schwefelylids der Formel (III b)

$(CH_3)_2SCH_2$ (III b)

zu einer Verbindung der Formel (IV)

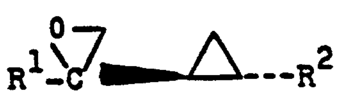

(IV)

umsetzt und anschließend die Verbindung der Formel (IV) ohne Isolierung der erhaltenen Verbindung der Formel (IV) durch Zugabe einer Verbindung der Formel (V)

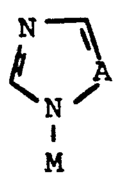

zum Reaktionsgemisch zur Verbindung der Formel (I) umsetzt und diese in Form der freien Base oder eines Säureadditionssalzes oder eines physiologisch hydrolysierbaren und akzeptablen Derivat isoliert.

2. Verfahren zum Herstellen einer Verbindung der Formel I gemäß Anspruch 1, dadurch gekennzeichnet, daß $R^1$ und $R^2$ jeweils gleich oder verschieden sind und unsubstituiertes Biphenyl, Phenoxyphenyl, Benzylphenyl, Benzyloxyphenyl, Naphthyl, Thienyl, Furyl oder Pyridyl oder eine unsubstituierte oder mit einem oder zwei Substituenten versehene Phenylgruppe bedeuten und diese Substituenten jeweils Fluor, Chlor, Brom, $(C_1-C_4)$-Alkyl, Trifluormethyl, $(C_1-C_4)$-Alkoxy, $(C_1-C_4)$-Alkylthio oder Nitro bedeuten.

3. Verfahren gemäß mindestens einem der Ansprüche 1 und 2, dadurch gekennzeichnet, daß $R^1$ und $R^2$ die Phenylgruppe bedeutet, welche unsubstituiert oder durch einen oder zwei Substituenten substituiert ist, die Fluor, Chlor, Brom oder die Trifluormethylgruppe bedeuten.

4. Verfahren gemäß mindestens einem der Ansprüche 1 bis 3, dadurch gekennzeichnet,daß die Substituenten von $R^1$ und $R^2$ jeweils Fluor oder Chlor bedeuten.

5. Verfahren zur Herstellung einer pharmazeutischen Zubereitung, dadurch gekennzeichnet, daß man eine Verbindung der Formel (I) oder eines ihrer Salze, gegebenenfalls zusammen mit einem üblichen pharmazeutischen Trägerstoff und/oder Hilfsstoff, in eine für pharmazeutische Zwecke geeignete Darreichungsform bringt.

6. Verfahren zur Herstellung eines fungiziden Mittels dadurch gekennzeichnet, daß man eine Verbindung der Formel (I) gemäß Anspruch 1 mit festen oder flüssigen Trägerstoffen sowie gegebenenfalls mit einem oder mehreren oberflächenaktiven Mitteln mischt.